(19) Europäisches Patentamt
European Patent Office
Office européen des brevets

(11) EP 2 990 408 B1

(12) **EUROPEAN PATENT SPECIFICATION**

(45) Date of publication and mention of the grant of the patent:
**18.09.2019 Bulletin 2019/38**

(21) Application number: **13882795.1**

(22) Date of filing: **23.04.2013**

(51) Int Cl.:
***C07F 9/655*** *(2006.01)* ***A61K 8/55*** *(2006.01)*
***A61K 31/661*** *(2006.01)* ***A61K 31/6615*** *(2006.01)*
***A61P 17/00*** *(2006.01)* ***A61P 17/16*** *(2006.01)*
***A61P 43/00*** *(2006.01)* ***A61Q 19/02*** *(2006.01)*

(86) International application number:
**PCT/JP2013/061848**

(87) International publication number:
**WO 2014/174583 (30.10.2014 Gazette 2014/44)**

(54) **NOVEL WHITENING AGENT**

NEUARTIGES BLEICHMITTEL

NOUVEL AGENT DE BLANCHIMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**02.03.2016 Bulletin 2016/09**

(73) Proprietor: **Nichirei Biosciences Inc.**
**Tokyo 104-8402 (JP)**

(72) Inventors:
- **NAGAMINE, Kenichi**
  **Tokyo 104-8402 (JP)**
- **TANABE, Hideya**
  **Higashimurayama-shi**
  **Tokyo 189-0003 (JP)**
- **HIMONO, Ayaka**
  **Higashimurayama-shi**
  **Tokyo 189-0003 (JP)**
- **FUJIWARA, Yoshihisa**
  **Ibaraki-shi**
  **Osaka 567-0085 (JP)**
- **ITO, Satoshi**
  **Ibaraki-shi**
  **Osaka 567-0085 (JP)**

(74) Representative: **Cabinet Plasseraud**
**66, rue de la Chaussée d'Antin**
**75440 Paris Cedex 09 (FR)**

(56) References cited:
**WO-A1-2012/098664**
**JP-A- S 533 538**
**JP-A- H02 279 690**
**JP-A- S59 157 009**
**JP-A- 2004 002 359**
**JP-B- S4 431 237**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### TECHNICAL FIELD

The present invention relates to a novel compound, which is a 3-hydroxy-2-pyrone derivative, and a skin-whitening agent and a melanin production inhibitor comprising such compound as an active ingredient.

### BACKGROUND ART

In general, pigmentation on the skin, such as blemishes, is considered to occur with the activation of melanocytes caused by ultraviolet stimulation and enhanced biosynthesis of melanin pigments. Accordingly, many compounds having inhibitory activity on the biosynthesis of melanin pigments (which, hereafter, may be referred to as "melanin production inhibitory activity") have been developed as active ingredients of skin-whitening agents.

Ascorbic acid inhibits melanin production in cells due to, for example, inhibitory effects on tyrosinase activity. It is well known that ascorbic acid can exert skin-whitening effects through melanin production inhibitory activity. However, ascorbic acid is disadvantageously degraded in an aqueous solution and causes coloration, such as browning. In order to overcome such disadvantages, many ascorbic acid derivatives that are more stable in aqueous solutions have been developed.

Examples of such ascorbic acid derivatives include esterified derivatives resulting from ester binding of a phosphate group or a fatty acid group such as stearic acid to a hydroxyl group at position 2, 3, 5, or 6 of the ascorbic acid and derivatives resulting from glycoside binding of sugar such as glucose thereto. For example, JP S44-31237 B (1969) describes a method for producing a cosmetic product comprising phosphorylated ascorbic acid or salt thereof. Such derivatives are considered to function in a manner such that they would be absorbed by a living body and hydrolyzed with enzymes to generate ascorbic acid. Accordingly, such derivatives are expected to exert skin-whitening effects in a living body.

Examples of other known compounds that can be used as active ingredients of skin-whitening agents include hydroquinone derivatives, kojic acid and derivatives thereof, and pyrone compounds and derivatives thereof (JP S53-3538 A (1978) and JP S59-157009 A (1984)). For example, JP S53-3538 A (1978) describes a skin-whitening agent comprising pyrone compounds. In JP S53-3538 A (1978), examples of pyrone compounds include kojic acid and comenic acid.

A pyrone compound; i.e., 3-hydroxy-2-pyrone (hereafter, it may occasionally be referred to as "3H2P"), is an oxidative degradation product of ascorbic acid. JP 2007-246475 A describes a method of using ascorbic acid as a starting material so as to synthesize 3H2P via oxidative reaction. In recent years, biological activity and chemical properties of 3H2P have been elucidated, and various applications of such compounds have been developed. For example, JP 2007-246475 describes that 3H2P is an amphipathic antioxidant, 3H2P is excellent in terms of inhibition of platelet aggregation and curing of hepatic disorders, and thus 3H2P can be used for a pharmaceutical composition or functional food or beverage product. JP H3-47899 A (1991) describes an aromatic composition comprising 3H2P.

The present inventors have discovered that 3H2P has sufficient inhibitory effects on tyrosinase activity and inhibitory effects on melanin production and thus would be useful as a skin-whitening ingredient (WO 2012/098664). WO 2012/098664 describes a skin-whitening agent, a tyrosinase activity inhibitor and a melanin production inhibitor comprising 3H2P.

### SUMMARY OF THE INVENTION

#### Object to Be Attained by the Invention

Known compounds that can be used as active ingredients of skin-whitening agents as described above have been problematic in several respects. For example, ascorbic acid derivatives are low in solubility in various solvents and they cannot exert satisfactory skin-whitening effects unless they are degraded in a living body. In addition to low solubility in various solvents, the skin-whitening effects of hydroquinone derivatives and kojic acid and derivatives thereof are not sufficient.

3H2P, which is an active ingredient of a skin-whitening agent developed by the present inventors, is high in solubility in various solvents and exerts satisfactory skin-whitening effects. However, 3H2P is disadvantageous in terms of its low stability in aqueous solutions.

Accordingly, it is an object of the present invention to provide a novel derivative of 3H2P with improved stability in aqueous solutions along with the retention of water solubility and skin-whitening effects.

Means for Attaining the Object

The present inventors have conducted concentrated studies in order to attain the above object. As a result, the present inventors have discovered that stability of a 3H2P derivative in aqueous solutions could be improved via ester binding of a phosphoric acid group to a hydroxyl group at position 3 of 3H2P. This has led to the completion of the present invention.

Specifically, the present invention is summarized as described below.

(1) A compound represented by formula (I-ii):

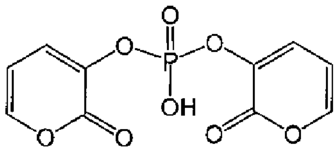

(I-ii)

or salt thereof.

(2) A skin-whitening agent comprising the compound or salt thereof according to (1) as an active ingredient.

(3) A melanin production inhibitor comprising the compound or salt thereof according to (1) as an active ingredient.

(4) A composition for an external skin preparation comprising the compound or salt thereof according to (1) and a component that is acceptable for application to the skin.

(5) A method for producing the compound or salt thereof according to (1) comprising a step of phosphorylation through the subjecting of 3-hydroxy-2-pyrone to phosphorylation so as to form a compound represented by Formula (I-ii) or salt thereof.

Effects of the Invention

The present invention can provide a novel derivative of 3H2P with improved stability in aqueous solutions along with the retention of water solubility and skin-whitening effects.

BRIEF DESCRIPTION OF THE DRAWINGS

Fig. 1 shows a correlation between test compound concentration, and the amount of melanin produced by melanoma cells and melanoma cell counts. A shows the test results obtained with the use of 3-hydroxy-2-pyrone phosphate monoester (not according to the invention) and 3-hydroxy-2-pyrone phosphate bisester (according to the invention) in the form of free acid as test compounds; and B shows the test results obtained with the use of a triethylamine salt of 3-hydroxy-2-pyrone phosphate monoester (not according to the invention) and a triethylamine salt of 3-hydroxy-2-pyrone phosphate bisester (according to the invention) as test compounds. Horizontal axis: test compound concentration (μg/ml); left axis: melanin amount; right axis: cell count. The melanin amount and the cell count of test groups to which the test compounds had been applied are represented relative to the measured melanin amount and cell count of the control group to which no test compound had been added (concentration: 0), which is designated as 100. "3H2P" indicates 3-hydroxy-2-pyrone, "3H2P-PME" indicates 3H2P phosphate monoester in the form of free acid, "3H2P-PBE" indicates 3H2P phosphate bisester in the form of free acid, "3H2P-PME $Et_3N$" indicates a triethylamine salt of 3H2P phosphate monoester, and "3H2P-PBE $Et_3N$" indicates a triethylamine salt of 3H2P phosphate bisester.

Fig. 2 shows the correlation between the test compound concentration and the inhibition rate of tyrosinase activity. "3H2P" indicates 3-hydroxy-2-pyrone, "3H2P-PME" indicates 3H2P phosphate monoester in the form of free acid, and "3H2P-PBE" indicates 3H2P phosphate bisester in the form of free acid.

EMBODIMENTS FOR CARRYING OUT THE INVENTION

<1: Novel compound>

The present invention relates to a compound represented by Formula (I-ii):

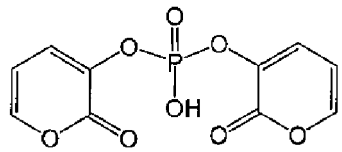

(I-ii)

or salt thereof.

The present inventors screened for a derivative of 3-hydroxy-2-pyrone (3H2P) exhibiting satisfactory stability in an aqueous solution from derivatives of such compounds. As a result, they have discovered a novel compound represented by Formula (I-ii) that is such a 3H2P derivative exhibiting high stability in an aqueous solution, in addition to high water solubility and high skin-whitening effects.

In this description, a compound represented by Formula (I-ii) is occasionally referred to as 3H2P phosphate bisester, respectively. Because of the structure shown above, it is possible for the compound represented by Formula (I-ii) according to the present invention to be more stably present in an aqueous solution than 3H2P.

The compound represented by Formula (I-ii) according to the present invention also includes a salt of such compound. Preferable examples of counterions of salts of the compound according to the present invention include, but are not limited to, monovalent, divalent, and trivalent metal cations and organic cations. More specifically, metal cations, such as sodium ions, potassium ions, calcium ions, and magnesium ions, and organic cations, such as trialkylamine ions (trialkylammonium ions) (e.g., trimethylamine ions (triethylammonium ions)), pyridinium ions, pyrrolidium ions, phosphonium ions, and sulfonium ions, are preferable. When the compound of the present invention forms a salt with the counterion, such compound can be used without substantial deterioration of skin-whitening effects such as melanin production inhibitory activity or deterioration of stability in aqueous solutions or solubility in water.

The compound represented by Formula (I-ii) according to the present invention also includes a solvate of such compound. Preferable examples of solvents that can form solvates with the compound of the present invention include, but are not limited to, water and organic solvents, such as methanol, ethanol, 2-propanol (isopropyl alcohol), and dimethyl sulfoxide (DMSO), with water being more preferable. When the compound of the present invention is in the form of a solvate with the solvent (e.g., a hydrate), such compound can be used without substantial deterioration of skin-whitening effects such as melanin production inhibitory activity or deterioration of stability in aqueous solutions or solubility in water.

Because of the features described above, the compound represented by Formula (I-ii) can exert satisfactory skin-whitening effects.

<2: Method for producing novel compound>

The present invention also relates to a method for producing a compound represented by Formula (I-ii) or salt thereof. Hereafter, preferable embodiments of the method according to the present invention are described in detail.

[2-1: Step of phosphorylation]

It is necessary that the method of the present invention comprise a step of phosphorylation through the subjecting of 3-hydroxy-2-pyrone (3H2P) to phosphorylation so as to form a compound represented by Formula (I-ii) or salt thereof.

In this step, 3H2P that is used as a starting material may be prepared by a known technique as described in, for example, JP 2007-246475 A, JP H3-47899 A (1991), or WO 2012/098664. Alternatively, 3H2P that has been prepared in advance may be purchased. The method of the present invention encompasses both embodiments.

In this step, 3H2P can be phosphorylated with the use of a reaction agent or reaction reagent that is generally used for phosphorylation of an alcohol compound in the art. An example of such reaction agent is phosphoryl chloride ($POCl_3$). When $POCl_3$ is used as a reaction agent, it is preferable that the step be performed in the presence of a proton scavenger, such as N,N,N',N'-tetramethyl-1,8-naphthalenediamine (Proton sponge®), so as to neutralize HCl generated upon the reaction. By performing this step with the use of such reaction agent, 3H2P can be efficiently phosphorylated, so as to obtain the compound represented by Formula (I-ii).

In the step of phosphorylation, it is preferable that 3H2P be allowed to react with a reaction agent in an adequate solvent. In such a case, the step of phosphorylation proceeds in a reaction system containing 3H2P, a reaction agent, and a solvent. Examples of solvents include trimethyl phosphate, ether-based solvents such as diethyl ether, tetrahydrofran (THF) and 1,4-dioxane, and toluene. By performing the step of phosphorylation in such solvent, the yield of the compound represented by Formula (I-ii) can be increased.

In the step of phosphorylation, 3H2P is preferably phosphorylated at a reaction temperature from -20°C to 40°C, and more preferably at room temperature (e.g., 20°C to 30°C). In addition, 3H2P phosphorylation is preferably performed for 10 minutes to 24 hours, and more preferably for about 2 hours (e.g., 1 to 3 hours). By performing the step of phosphorylation under the conditions described above, the yield of the compound represented by Formula (I-ii) can

be increased.

As a result of the step of phosphorylation, a compound represented by Formula (I-ii) (i.e., 3H2P phosphate bisester) is generated. 3H2P phosphate bisester comprises dissociable group per molecule. Thus, the compound represented by Formula (I-ii) can be hydrolyzed by an acid originating from its dissociable group. Accordingly, it is preferable that the step of phosphorylation be carried out in the presence of an adequate base, in addition to 3H2P and a reaction agent. When the step of phosphorylation is carried out in the presence of a base, the compound represented by Formula (I-ii) forms a salt with such base. Examples of such bases include organic bases and inorganic bases, such as triethylammonium bicarbonate (TEAB), sodium bicarbonate, and sodium carbonate. When the phosphorylation reaction is initiated in this step, the base may be added to a reaction system containing 3H2P, a reaction agent, and, optionally a solvent. Alternatively, the base may be added to the reaction system after the completion or during the phosphorylation reaction. By performing the step in the presence of the base, a reduction in the yield of the compound represented by Formula (I-ii) caused by acid hydrolysis can be substantially suppressed.

<2-2: Step of purification>

The method of the present invention can optionally comprise a step of purification of the compound represented by Formula (I-ii) or salt thereof obtained by the step of phosphorylation. As a result of the step of phosphorylation, a compound represented by Formula (I-ii) is generated. This step is aimed at purifying at least one of the compound represented by Formula (I-ii), and a salt thereof from the reaction product obtained in the step of phosphorylation as the compound represented by Formula (I-ii) or salt thereof.

In the step of purification, the means of purifying the compound represented by Formula (I-ii) or salt thereof is not particularly limited. Various means of separation and purification that are generally used in the art, such as various chromatography and high-performance liquid chromatography (HPLC) techniques, including adsorption, ion-exchange, gel filtration or reversed-phase, solvent fractionation, or recrystallization, can be employed. Thus, at least one of the compound represented by Formula (I-ii), and a salt thereof can be purified as the compound represented by Formula (I-ii) or salt thereof.

When a base is used in the step of phosphorylation, the compound represented by Formula (I-ii) forms a salt with such base. In such a case, a salt of the compound represented by Formula (I-ii) may be treated with an adequate cation-exchange means, so that the salt can be converted into free acid by the ion exchange means. The cation-exchange means is not particularly limited, provided that it is a strongly acidic cation-exchange resin generally used in the art. An example of the cation-exchange means is a strongly acidic cation-exchange resin in sulfonic acid form (e.g., Dowex® 88). With the use of the cation-exchange means, the compound represented by Formula (I-ii) in the form of free acid can be obtained.

<3: Application of novel compound>

The present invention also relates to a skin-whitening agent or a melanin production inhibitor comprising, as an active ingredient, the compound represented by Formula (I-ii) or salt thereof.

The compound represented by Formula (I-ii) of the present invention can inhibit melanin production and/or tyrosinase activity. The compound represented by Formula (I-ii) can exert satisfactory skin-whitening effects because of the features described above.

The compound represented by Formula (I-ii) contained, as an active ingredient, in the skin-whitening agent or the melanin production inhibitor of the present invention may be in the form of a salt or solvate of such compound. A salt of the compound represented by Formula (I-ii) is preferably a physiologically acceptable salt as described above (e.g., a salt that is acceptable as a pharmaceutical, cosmetic, or food or beverage product). A solvate of the compound represented by Formula (I-ii) is preferably a physiologically acceptable solvate as described above (e.g., a solvate that is acceptable as a pharmaceutical, cosmetic, or food or beverage product). When the compound represented by Formula (I-ii) is in the form of a salt or solvate, such compound can exert satisfactory skin-whitening effects along with the retention of high stability in an aqueous solution and high solubility in water.

The compound represented by Formula (I-ii) of the present invention can be incorporated as an active ingredient exerting skin-whitening effects, inhibitory effects on melanin production, or inhibitory effects on tyrosinase activity into an external skin preparation, such as a cosmetic product, quasi-drug, or pharmaceutical product, together with a component that is acceptable for application to the skin (e.g., another component exerting skin-whitening effects, an active ingredient exerting other effects, or a component used for formulation). Accordingly, the present invention also relates to a composition for an external skin preparation comprising the compound represented by Formula (I-ii) or salt thereof and a component that is acceptable for application to the skin. The amount of the compound represented by Formula (I-ii) in the composition for an external skin preparation is not particularly limited, provided that it is an effective amount for the exertion of skin-whitening effects, inhibitory effects on melanin production, and/or inhibitory effects on tyrosinase

activity. On the basis of the results shown in Use Example III-3 and an amount of active ingredients that can be incorporated into a general external skin preparation, for example, the amount of the compound is preferably 0.0001% to 5% by weight relative to the total weight of the composition for an external skin preparation. Thus, the composition for an external skin preparation of the present invention can exert skin-whitening effects, inhibitory effects on melanin production, and/or inhibitory effects on tyrosinase activity of interest.

Examples of other ingredients exerting skin-whitening effects that can be incorporated into the composition for an external skin preparation of the present invention include ascorbic acid, an ascorbic acid derivative (e.g., a salt of an ascorbic acid phosphate ester, a salt of an ascorbic acid sulfate ester, ascorbic acid glucoside, or ethyl ascorbate), a γ-pyrone glycoside, γ-pyrones such as kojic acid and a kojic acid derivative, and a hydroquinone derivative such as arbutin.

Examples of components for formulation that can be incorporated into the composition for an external skin preparation of the present invention include components that are generally used for the formulation of cosmetic products, quasi-drugs, or pharmaceutical products, such as purified water, alcohols, oil substances, moisturizers, thickeners, preservatives, emulsifiers, crude drug components, ultraviolet absorbers, pigments, aromas, emulsion stabilizers, and pH adjusters.

The form of the composition for an external skin preparation of the present invention is not particularly limited. Any form that is generally used in the art, such as a cream, ointment, emulsion, lotion, solution, gel, facial mask, or stick, can be adequately selected.

Because of the features described above, the composition for an external skin preparation of the present invention can exert skin-whitening effects, inhibitory effects on melanin production, and/or inhibitory effects on tyrosinase activity of interest.

Through administration of the compound represented by Formula (I-ii) or the composition for an external skin preparation of the present invention to a subject, such as a human, who desires skin-whitening, skin-whitening can be realized (i.e., skin damage caused by pigmentation, such as blemishes or freckles, can be substantially inhibited or relieved). Accordingly, the present invention relates to a method for skin-whitening comprising a step of administering the compound represented by Formula (I-ii) or salt thereof to a subject, such as a human, who desires skin-whitening. Also, the present invention relates to use of the compound represented by Formula (I-ii) or salt thereof in the manufacture of an external skin preparation or a medicament for skin-whitening purposes. The present invention further relates to a compound represented by Formula (I-ii) or salt thereof for use in skin-whitening applications. The method includes administration of the compound represented by Formula (I-ii) or the composition for an external skin preparation to a subject, such as a human, before pigmentation on the skin occurs due to sunburn, so as to prevent the pigmentation on the skin or suppress the progression thereof. Alternatively, the compound represented by Formula (I-ii) or the composition for an external skin preparation may be administered to a subject who has already experienced pigmentation, so as to relieve the skin damage caused by pigmentation. Such method may be implemented for a cosmetic purpose, or it may be implemented by a physician for a medical purpose.

The compound represented by Formula (I-ii) of the present invention can be used for a method for the inhibition of melanin production *in vivo* or *in vitro*. Accordingly, the present invention relates to a method for the inhibition of melanin pigment production *in vivo* or *in vitro* comprising a step of administering the compound represented by Formula (I-ii) or salt thereof to cells of an animal, such as a human, *in vivo* or *in vitro*. In this description, the term "inhibition of melanin pigment production *in vivo* (or inhibition of melanin production)" refers to inhibition of melanin pigment production in a living body such as a human (e.g., inhibition of melanin pigment production in skin cells). Such method may be implemented for a cosmetic purpose, or it may be implemented by a physician for a medical purpose.

The compound represented by Formula (I-ii) or the composition for an external skin preparation of the present invention can be used for the prevention or treatment of a disease or symptom that is relieved by the inhibition of melanin production. Accordingly, the present disclosure relates to a method for the prevention or treatment of a disease or symptom that is relieved by the inhibition of melanin pigment production comprising a step of administering the compound represented by Formula (I-ii) or salt thereof to a subject, such as a human, who is in need of prevention or treatment of such disease or symptom. The present disclosure also relates to use of the compound represented by Formula (I-ii) or salt thereof in the manufacture of an external skin preparation for the inhibition of melanin production, or of a medicament for the prevention or treatment of a disease or symptom that is relieved by inhibition of melanin pigment production in cells. Further, the present invention relates to a compound represented by Formula (I-ii) or salt thereof for use in the inhibition of melanin pigment production by cells *in vivo* or *in vitro*. Examples of such disease or symptom include blemishes and freckles.

Tyrosinase is an important enzyme that acts when a melanin pigment is produced from tyrosine as an amino acid. Many known compounds having skin-whitening effects have inhibitory activity on tyrosinase. The compound represented by Formula (I-ii) of the present invention can be used in a method for the inhibition of tyrosinase activity *in vivo* or *in vitro*. Accordingly, the present invention also relates to a method of inhibiting tyrosinase activity *in vivo* or *in vitro* comprising a step of applying the compound represented by Formula (I-ii) or salt thereof to tyrosinase *in vivo* or *in vitro*. In this description, the term "inhibition of tyrosinase activity *in vivo* (or inhibition of tyrosinase)" refers to inhibition of

tyrosinase activity in a living body such as a human (e.g., inhibition of tyrosinase activity in skin cells). Such method may be implemented for a cosmetic purpose, or it may be implemented by a physician for a medical purpose.

The compound represented by Formula (I-ii) or the composition for an external skin preparation of the present invention can be used for the prevention or treatment of a disease or symptom that is relieved by the inhibition of tyrosinase activity. Accordingly, the present disclosure relates to a method for the prevention or treatment of a disease or symptom that is relieved by the inhibition of tyrosinase activity comprising a step of administering the compound represented by Formula (I-ii) or salt thereof to a subject, such as a human, who is in need of prevention or treatment of such disease or symptom. The present disclosure also relates to use of the compound represented by Formula (I-ii) or salt thereof in the manufacture of an external skin preparation for the inhibition of tyrosinase activity, or of a medicament for the prevention or treatment of a disease or symptom that is relieved by inhibition of tyrosinase activity. Further, the present invention relates to a compound represented by Formula (I-ii) or salt thereof for use in the inhibition of tyrosinase activity *in vivo* or *in vitro.* Examples of such disease or symptom include blemishes and freckles.

The forms of administration of the compound represented by Formula (I-ii) to a subject, such as a human, for use in the skin-whitening or for use in the purpose of inhibition of melanin production or tyrosinase activity *in vivo* are not particularly limited. For example, the compound represented by Formula (I-ii) may be administered to the subject via percutaneous administration involving direct application thereof to the target skin (for example, in the form of the external skin preparations), oral administration (for example, in the form of powder, tablets, granules, fine grains, liquid, capsules, pills, troches, peroral liquid preparations, suspensions, emulsions, syrups, or elixirs), or parenteral administration other than percutaneous administration (for example, in the form of injection preparations, instillation, suppositories, inhalants, or transmucosal absorbents).

EXAMPLES

Hereafter, the present invention is described in greater detail with reference to the examples, although the technical scope of the present invention is not limited to these examples.

<I: Synthesis of 3H2P phosphate ester>

[I-1: Synthesis]

3-Hydroxy-2-pyrone (3H2P) (100 mg), 287 mg of Proton sponge® (Aldrich), and 3 ml of trimethyl phosphate were added into a 50-ml test tube with a ground-glass joint under a nitrogen stream, so as to prepare a solution. The resulting solution was cooled to 0°C, and 63 μl of $POCl_3$ was added to the solution under ice cooling (0°C) little by little. The resulting reaction solution was slowly brought back to room temperature and it was then agitated overnight. An aqueous solution of 0.1 M triethylammonium bicarbonate (TEAB) (4 ml) was added to the reaction solution, and the mixture was agitated at room temperature for 2 hours. The reaction was carried out 5 times in the manner described above by modifying the amounts of starting materials used and the reaction conditions (e.g., reaction temperature and/or duration). The reaction solutions obtained by all the reactions were integrated into a single solution. The resulting solution was mounted on a DEAE column (DEAE TOYOPEARL® 650 M; about 150 ml of gel; Tosoh Corporation), and a non-adsorbed fraction containing unreacted 3H2P and Proton sponge was eluted with water. Thereafter, the adsorbed fraction was eluted from the DEAE column under gradient elution conditions from water (A) to an aqueous solution of 0.35 M TEAB (B) (a concentration gradient of the solution B from 0% to 100% for 0 to 300 minutes). By observing UV absorption of the eluate, a fraction of the product exhibiting UV absorption was separated and concentrated. The fraction of the product was mounted on a strongly acidic cation-exchange resin column (Dowex® 88; about 100 ml of gel; Dow Chemical Co.) and eluted with water. By observing UV absorption of the eluate, a fraction of the product exhibiting UV absorption was separated. The obtained fraction was lyophilized, and 864 mg of 3H2P phosphate monoester (1) in the form of free acid and 1118.4 mg of 3H2P phosphate bisester (2) in the form of free acid were obtained.

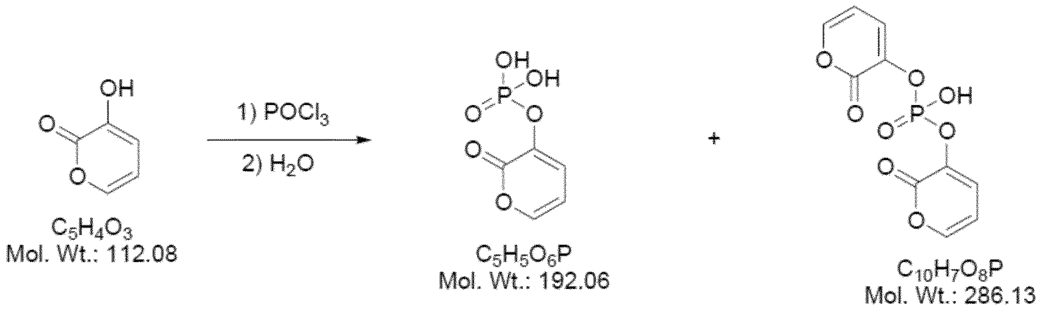


3H2P 3H2P phosphate monoester (1) 3H2P phosphate bisester (2)

[1-2: Instrumental analysis of product]

The product was subjected to instrumental analysis under the conditions described below.

[LC-MS analysis]

- MS conditions

Apparatus: micro TOF Focus (Bruker Daltonics)
Ionization: ESI (positive ion-low mode; negative ion-low mode)

- LC conditions

Apparatus: Agilent 1100 Series
Column:

Agilent Zorbax SB-C18 (Agilent Technologies)
2.1 mm (inner diameter) × 150 mm (column length) (particle size: 3.5 μm)

Eluate:

(A) Acetonitrile
(B) Aqueous solution of 10 mM $NH_4HCO_3$

Gradient: Concentration gradient of solution A from 0% to 100% in 0 to 15 minutes
Flow rate: 0.2 ml/min
Sample: 1,000 ppm (diluted with aqueous solution of 10 mM $NH_4HCO_3$)
Amount of injection: 2 μl
Column temperature: 40°C
Detector: Multi-channel detector (UV: 293 nm, 295 nm)

[$^1$H-NMR analysis]

Apparatus: JEOL AL-400 (JEOL, Ltd.)
Resonance frequency: 395.75 MHz
Solvent: $D_2O$
Internal standard: water (4.81 ppm)

[$^{13}C$-NMR analysis]

Apparatus: JEOL AL-400 (JEOL, Ltd.)
Resonance frequency: 99.45 MHz
Solvent: $D_2O$
External standard: tetramethylsilane ($CDCl_3$ solution) (0 ppm)

[$^{31}P$-NMR analysis]

Apparatus: JEOL AL-400 (JEOL, Ltd.)
Resonance frequency: 160.10 MHz
Solvent: $D_2O$
External standard: 85% $H_3PO_4$(0 ppm)

[Results of analysis]

3H2P phosphate monoester (1) (free acid):

HRMS calculated: $C_5H_6O_6P$ 192.9897; measured: 192.9886 $(M+H)^+$, calculated: $C_5H_4O_6P$ 190.9751; measured: 190.9743 $(M-H)^-$; $^1H$-NMR ($D_2O$, 395.75 MHz) $\delta$ ppm: 7.40 (ddd, $J_{HH}$ = 5.1 Hz, $J_{HH}$ = 1.7 Hz, $J_{HP}$ = 1.0 Hz, 1 H), 7.28 (ddd, $J_{HH}$ = 7.3-7.5 Hz, $J_{HH}$ = 1.7 - 1.9 Hz, $J_{HP}$ = 1.7 - 1.9 Hz, 1 H), 6.36 (dd, $J_{HH}$ = 7.3 Hz, $J_{HH}$ = 5.1 Hz, 1 H); $^{13}C$-NMR ($D_2O$, 99.45 MHz) $\delta$ ppm: 162.4 ($J_{CP}$ = 6.6 Hz), 147.5 ($J_{CP}$ = 1.6 Hz), 138.1 ($J_{CP}$ = 6.6 Hz), 128.9 ($J_{CP}$ = 4.1 Hz), 107.2 ($J_{CP}$ = 1.6 Hz); $^{31}P$-NMR ($D_2O$, 160.10 MHz) $\delta$ ppm: -3.5

3H2P phosphate bisester (2) (free acid):

HRMS (MALDI-TOF) calculated: $C_{10}H_8O_8P$ 286.9951; measured: 286.9947 $(M+H)^+$, calculated: $C_{10}H_6O_8P$ 284.9806; measured: 284.9805 $(M-H)^-$; $^1H$-NMR ($D_2O$, 395.75 MHz) $\delta$ ppm: 7.55 (ddd, $J_{HH}$ = 5.1 Hz, $J_{HH}$ = 1.5 - 1.7 Hz, $J_{HP}$ = 1.0 - 1.2 Hz, 1 H), 7.48 (ddd, $J_{HH}$ = 7.3-7.5 Hz, $J_{HH}$ = 1.7 - 1.9 Hz, $J_{HP}$ = 1.7 - 1.9 Hz, 1 H), 6.50 (dd, $J_{HH}$ = 7.3 Hz, $J_{HH}$ = 5.1 Hz, 1 H); $^{13}C$-NMR ($D_2O$, 99.45 MHz) $\delta$ ppm: 162.0 ($J_{CP}$ = 6.6 Hz), 147.8 ($J_{CP}$ = 1.6 Hz), 137.9 ($J_{CP}$ = 7.4 Hz), 129.5 ($J_{CP}$ = 4.1 Hz), 107.0 ($J_{CP}$ = 1.6 Hz); $^{31}P$-NMR ($D_2O$, 160.10 MHz) $\delta$ ppm: -8.4

<II: Synthesis of 3H2P phosphate ester salt>

[II-1: Synthesis]

3-Hydroxy-2-pyrone (3H2P) (500 mg, 4.46 mmol), 1.14 g (5.31 mmol) of Proton sponge® (Aldrich), and 5 ml of trimethyl phosphate were added into a 50-ml test tube with a ground-glass joint under a nitrogen stream, so as to prepare a solution. The resulting solution was cooled to 0°C, and 499 µl (5.35 mmol) of $POCl_3$ was added to the solution under ice cooling (0°C) little by little. The resulting reaction solution was agitated at the same temperature as described above for 2 hours. An aqueous solution of 0.1 M triethylammonium bicarbonate (TEAB) was added to the reaction solution, and the mixture was agitated at room temperature for 2 hours. The resulting reaction solution was diluted with 100 ml of water. The resulting solution was mounted on a DEAE column (DEAE TOYOPEARL® 650 M; about 150 ml of gel; Tosoh Corporation), and a fraction of the product was separated under gradient elution conditions from water (A) to an aqueous solution of 0.3 M TEAB (B) (a concentration gradient of the solution B from 0% to 100% for 0 to 40 minutes). The fraction of the product was mounted on a HPLC column (Phenomenex Luna® 10 µ C18 (2), 10 µm, 21.2 × 250 mm, Phenomenex), and the fraction of the product was separated under gradient elution conditions from an aqueous solution of 0.1 M TEAB (A) to acetonitrile (B) (a concentration gradient of the solution B from 0% to 40% for 0 to 30 minutes). The obtained fraction was lyophilized, and a triethylamine salt (1.5 equivalents) of 3H2P phosphate monoester (1) (138.6 mg, 10.6%) and a triethylamine salt (1 equivalent) of 3H2P phosphate bisester (2) (549.6 mg, 31.8%) were obtained (the yield was determined in terms of a monotriethylamine salt).

[II-2: Instrumental analysis of product]

The product was subjected to instrumental analysis under the conditions described below.

[LC-MS analysis]

Apparatus: Shimadzu LCMS-2010A EV (Shimadzu Corporation)
Column:

Agilent Zorbax SB-C18 (Agilent Technologies)
2.1 mm (inner diameter) × 150 mm (column length) (particle size: 3.5 μm)

Eluate:

(A)10 mM $NH_4HCO_3$ (in the case of 3H2P phosphate monoester)
10 mM $NH_4HCO_3$ + 0.1% $HCOOH/H_2O$
(in the case of 3H2P phosphate bisester)
(B) Acetonitrile

Gradient: Concentration gradient of solution B from 0% to 100% in 0 to 15 minutes
Flow rate: 0.2 ml/min
Diluent (concentration): water (1 mg/ml)
Amount of injection: 2 μl
Column temperature: 40°C
Detector 1: ESI
Probe voltage:

+1.6 kV (ESI-positive-ion-mode)
: -1.6 kV (ESI-negative-ion-mode)

CDL temperature: 250°C
Block heater temperature: 200°C
Nebulizer gas flow rate: 1.5 ml/min
Dry gas pressure: 0.02 MPa
Detector 2: PDA (190 to 800 nm)

[$^1$H-NMR analysis]

Apparatus: JEOL JNM-ECS400 (JEOL, Ltd.)
Resonance frequency: 400 MHz
Solvent: $D_2O$
Internal standard: 3-(trimethylsilyl)-1-propanesulfonic acid sodium salt

[Results of analysis]

3H2P phosphate monoester (1) (triethylamine salt, 1.5 equivalents):

ESI-MS m/z: 193 $(M+H)^+$, 191 $(M-H)^-$; $^1$H-NMR ($D_2O$, 400 MHz) δ ppm: 7.48 (d, $J_{HH}$ = 4.4 Hz, 1 H), 7.37 (m, 1 H), 6.47 (dd, $J_{HH}$ = 7.4 Hz, $J_{HH}$ = 5.1-5.2 Hz, 1 H), 3.2 (q, $J_{HH}$ = 7.4 Hz, 9H), 1.27 (t, $J_{HH}$ = 7.4 Hz, 13.5H).

3H2P phosphate bisester (2) (triethylamine salt, 1 equivalent):

ESI-MS m/z: 287 $(M+H)^+$, 285 $(M-H)^-$; $^1$H-NMR ($D_2O$, 400 MHz) δ ppm: 7.53 (d, $J_{HH}$ = 5.1 Hz, 2H), 7.47 (d, $J_{HH}$ = 7.4 Hz, 2H), 6.48 (dd, $J_{HH}$ = 7.3 Hz, $J_{HH}$ = 5.2 to 5.3 Hz, 2H), 3.19 (q, $J_{HH}$ = 7.4 Hz, 6H), 1.27 (t, $J_{HH}$ =7.4 Hz, 9H).

[III: Use example]

[III-1: Stability test]

3H2P phosphate monoester (1) (not according to the invention) and 3H2P phosphate bisester (2) (according to the invention) in the form of free acid were dissolved in a 20 mM citrate buffer solution (pH 6.0) to a concentration of 0.1% (weight/volume). The test solutions were stored at 4°C, 25°C, or 40°C for 3 weeks. 3H2P phosphate monoester (1) or 3H2P phosphate bisester (2) contained in the stored test solutions were quantitatively analyzed via reversed-phase HPLC (Inertsil® ODS-3, 5 μm, 4.6 × 250 mm, GL Sciences). For comparative example, the similar test was carried out in the manner described above with the use of 3H2P. Table 1 shows the percentages of compounds remaining in the test solutions after storage at relevant temperatures (i.e., the percentage on the basis of the amount of the compound when the test was initiated).

Table 1

| Compound | 4°C | 25°C | 40°C |
|---|---|---|---|
| 3H2P | 98.4 | 87.3 | 53.2 |
| 3H2P phosphate monoester (1) (not according to the invention) | 99.8 | 96.6 | 77.0 |
| 3H2P phosphate bisester (2) (according to the invention) | 100 | 100 | 99.3 |

As shown in Table 1, 3H2P phosphate monoester (1) and 3H2P phosphate bisester (2) were found to show remarkably improved stability in weak-acidic to neutral water, compared with non-phosphorylated 3H2P.

[III-2: Test for melanin production inhibition]

Mouse-derived B16 melanoma cells 4A5 (RIKEN) were seeded on a 6-well plate with the use of DMEM medium containing 10% FBS to a cell density of 5.0 × $10^4$ cells/well (day 0 of culture), and culture was conducted at 37°C in the presence of 5% $CO_2$ for 24 hours. On the following day, the cell-seeded medium was exchanged with a medium supplemented with the test compound at a given concentration (day 1 of culture). The medium was exchanged with a fresh medium every other day from day 2 and thereafter. The cells were recovered via treatment with trypsin on day 6 of culture, and melanin was extracted using an aqueous solution of 1 N sodium hydroxide. The absorbance of the melanin extract was measured at 405 nm, and the melanin amount was determined using a calibration curve prepared with the use of a melanin standard sample. In addition, the protein amount of the cells recovered in the manner described below was determined using the DC Protein Assay Kit (BIO-RAD) and employed as the cell count index. Fig. 1 shows the correlation of the test compound concentration, the melanin amount of the melanoma cells, and the melanoma cell count on the basis of the measured values described above. Fig. 1A shows the test results obtained with the use of 3H2P phosphate monoester (1) (not according to the invention) and 3H2P phosphate bisester (2) (according to the invention) in the form of free acid as test compounds; and Fig. 1B shows the test results obtained with the use of a triethylamine salt of 3H2P phosphate monoester (1) (not according to the invention) and a triethylamine salt of 3H2P phosphate bisester (2) (according to the invention) as test compounds. In the figure, the melanin amount and the cell count of test groups to which the test compounds had been added are represented relative to the measured melanin amount and cell count of the control group to which no test compound had been added (concentration: 0), which is designated as 100.

As shown in Fig. 1, 3H2P phosphate monoester (1) and 3H2P phosphate bisester (2) at concentrations of 1, 5, and 10 μg/ml exhibited melanin production inhibitory activity that was slightly lower than that of 3H2P but equivalent to that of arbutin as a known skin-whitening agent. At the concentration of 5 μg/ml, 3H2P phosphate monoester (1) and 3H2P phosphate bisester (2) inhibited melanin production to the level approximately 50% of the melanin amount of the control group. In addition, 3H2P phosphate monoester (1) and 3H2P phosphate bisester (2) in the form of free acid or a triethylamine salt exhibited approximately equivalent melanin production inhibitory activity.

[III-3: Test of tyrosinase activity inhibition]

A solution of 6 mM L-DOPA, an aqueous solution of the test compound adjusted to a given concentration, and a phosphate buffer (pH 6.8) were mixed at a ratio of 2:2:1 by volume. The resulting substrate mixture (1.0 ml) was mixed with 0.2 ml of a solution of 0.017 mg/ml tyrosinase (Sigma), and the enzyme reaction was initiated at 37°C. The absorbance of the enzyme reaction solution was measured at 475 nm 0 and 3 minutes after the initiation of the reaction. The test group subjected to the enzyme reaction in the same manner described above, except that the test compound was not

included, was designate as the blank test group. On the basis of the measured absorbance, the inhibition rate of tyrosinase activity was determined using the formula shown below. Fig. 2 shows the results.

$$\text{Inhibition rate of tyrosinase activity (\%)} = \left( 1 - \frac{\text{The difference in the absorbance of the test compound in the enzyme reaction solution between 0 minutes and 3 minutes after the initiation of the reaction}}{\text{The difference in the absorbance of the blank in the enzyme reaction solution between 0 min and 3 min}} \right) \times 100$$

As shown in Fig. 2, 3H2P phosphate monoester (1) (not according to the invention) and 3H2P phosphate bisester (2) (according to the invention) in the form of free acid exhibited low tyrosinase inhibitory activity at the concentration of 0.1 to 0.5 mg/ml. Tyrosinase inhibitory activity of 3H2P phosphate monoester (1) and that of 3H2P phosphate bisester (2) were lower than the tyrosinase inhibitory activity of 3H2P at the same concentration.

## Claims

1. A compound represented by formula (I-ii):

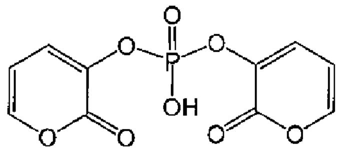

(I-ii)

or salt thereof.

2. A skin-whitening agent comprising the compound or salt thereof according to claim 1 as an active ingredient.

3. A melanin production inhibitor comprising the compound or salt thereof according to claim 1 as an active ingredient.

4. A composition for an external skin preparation comprising the compound or salt thereof according to claim 1 and a component that is acceptable for application to the skin.

5. A method for producing the compound or salt thereof according to claim 1 comprising a step of phosphorylation through the subjecting of 3-hydroxy-2-pyrone to phosphorylation so as to form a compound represented by Formula (I-ii) or salt thereof.

## Patentansprüche

1. Verbindung, dargestellt durch die Formel (I-ii):

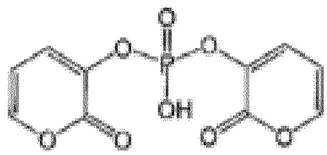

(I-ii)

oder ein Salz davon.

2. Hautaufhellungs-Mittel umfassend die Verbindung oder das Salz davon nach Anspruch 1 als einen Wirkstoff.

3. Inhibitor der Melamin-Herstellung, umfassend die Verbindung oder das Salz davon nach Anspruch 1 als einen

Wirkstoff.

**4.** Zusammensetzung für eine Außenhaut-Zubereitung, umfassend die Verbindung oder das Salz davon nach Anspruch 1 und eine Komponente, die zur Anwendung auf der Haut annehmbar ist.

**5.** Verfahren zur Herstellung der Verbindung oder des Salzes davon nach Anspruch 1, umfassend einen Phosphorylierungsschritt durch das Aussetzen von 3-Hydroxy-2-pyron gegenüber Phosphorylierung, um eine Verbindung, dargestellt durch die Formel (I-ii) oder ein Salz davon zu bilden.

## Revendications

**1.** Composé représenté par la formule (I-ii) :

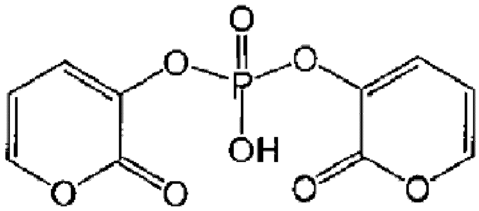

(I-ii)

ou un sel de celui-ci.

**2.** Agent de blanchiment de la peau comprenant le composé ou un sel de celui-ci selon la revendication 1 en tant qu'ingrédient actif.

**3.** Inhibiteur de production de mélanine comprenant le composé ou un sel de celui-ci selon la revendication 1 en tant qu'ingrédient actif.

**4.** Composition destinée à une préparation cutanée à usage externe comprenant le composé ou un sel de celui-ci selon la revendication 1 et un composé qui est acceptable pour une application sur la peau.

**5.** Procédé de production du composé ou d'un sel de celui-ci selon la revendication 1 comprenant une étape de phosphorylation par l'intermédiaire d'une soumission de 3-hydroxy-2-pyrone à une phosphorylation afin de former un composé représenté par la formule (I-ii) ou un sel de celui-ci.

Fig. 1

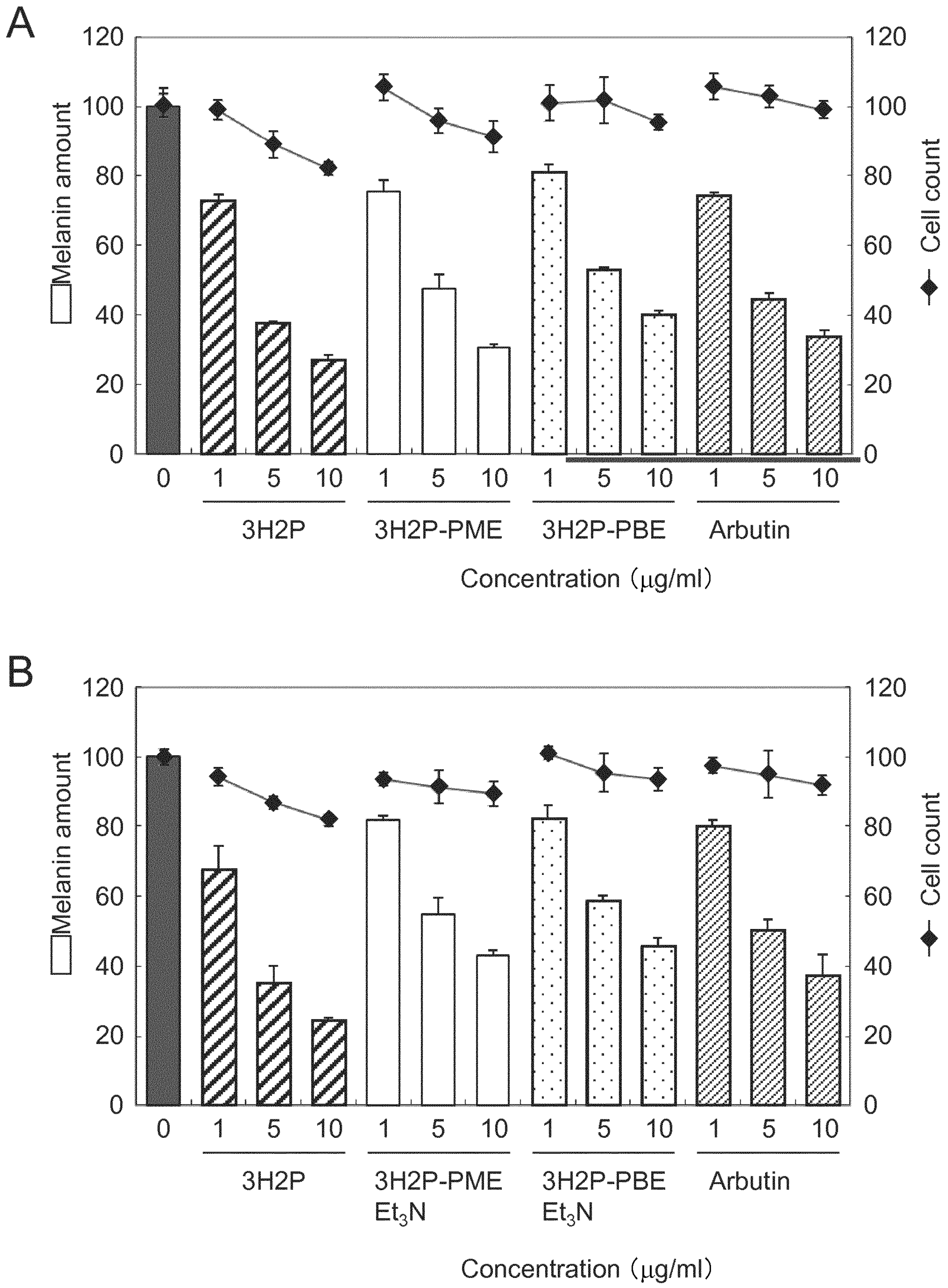
A
Melanin amount
Cell count
120
100
80
60
40
20
0
0
1
5
10
3H2P
3H2P-PME
3H2P-PBE
Arbutin
Concentration (μg/ml)
B
Melanin amount
Cell count
3H2P
3H2P-PME
$Et_3N$
3H2P-PBE
$Et_3N$
Arbutin
Concentration (μg/ml)

Fig. 2

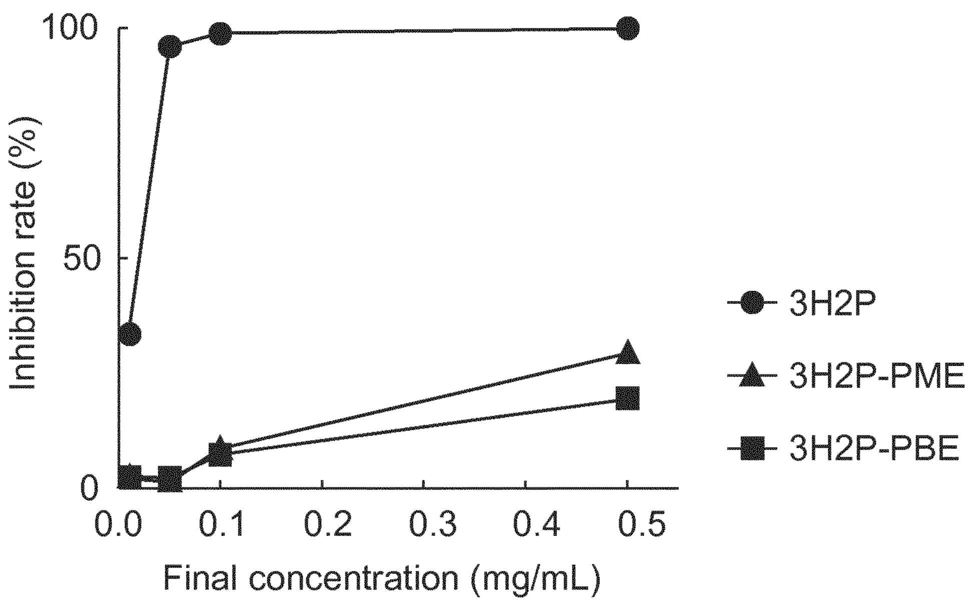
100
50
0
Inhibition rate (%)
0.0
0.1
0.2
0.3
0.4
0.5
Final concentration (mg/mL)
3H2P
3H2P-PME
3H2P-PBE

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP S4431237 B
- JP S533538 A
- JP S59157009 A
- JP 2007246475 A
- JP H347899 A
- WO 2012098664 A